(19) Europäisches Patentamt
European Patent Office
Office européen des brevets

(11) **EP 4 086 344 A1**

(12) **EUROPEAN PATENT APPLICATION**
published in accordance with Art. 153(4) EPC

(43) Date of publication:
**09.11.2022 Bulletin 2022/45**

(21) Application number: **20909311.1**

(22) Date of filing: **28.12.2020**

(51) International Patent Classification (IPC):
**C12N 15/10** (2006.01)    **C40B 50/06** (2006.01)

(52) Cooperative Patent Classification (CPC):
**C12N 15/10; C40B 50/06**

(86) International application number:
**PCT/CN2020/140247**

(87) International publication number:
**WO 2021/136194 (08.07.2021 Gazette 2021/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.12.2019   CN 201911389782**

(71) Applicant: **Nanjing GenScript Biotech Co., Ltd.
Nanjing, Jiangsu 211100 (CN)**

(72) Inventors:
• **DAI, Xiaohui
Nanjing, Jiangsu 211100 (CN)**
• **LI, Yifan
Nanjing, Jiangsu 211100 (CN)**
• **WU, Cheng-Hsien
Nanjing, Jiangsu 211100 (CN)**

(74) Representative: **Pharma Patents International AG
Leonhardsgraben 52
4051 Basel (CH)**

Remarks:
The complete document including Reference Table(s) and the Sequence Listing(s) can be downloaded from the EPO website

(54) **METHOD FOR CONSTRUCTING GENE MUTATION LIBRARY**

(57)   A method for constructing a gene mutation library, also relating to the gene mutation library obtained by the method, a kit for the method for constructing a gene mutation library, and a method for analyzing the relationships between amino acid mutations in a protein and the properties, regulation, and/or function of the protein using the gene mutation library constructed by the method.

**EP 4 086 344 A1**

**Description**

**Technical Field**

The present invention belongs to the field of biotechnology. Specifically, the present invention relates to the field of constructing a gene mutation library.

**Background Art**

A Gene mutation library is a combination of a large amount of DNA variant sequences and is a product of the combination of gene synthesis, gene mutation and directed evolution study. The gene mutation library has been increasingly used in research fields, such as high-throughput drug target screening, directed evolution by protein engineering, synthesizing diversity antibody libraries to screen variant antibodies with high-affinity and specificity, *etc.* The construction of a gene mutation library comprises using molecule directed evolution to simulate the natural selection process, thereby changing the amino acid sequence of the original protein and obtain a mutant protein with specific function.

Molecular directed evolution comprises using modern molecular biology methods to create a library comprising a large number of mutant homologous genes to artificially simulate the natural evolution mechanism and using sensitive directed screening strategies to create mutant proteins or other biomolecules that do not exist in nature or have significant changes in certain properties. Molecular directed evolution has been widely used in molecular modification of proteins and is considered to be the most efficient method for improving protein properties or regulating sequences.

The gene mutation library can be used to alter specific sites in proteins or regulatory regions in DNA, and can be used to study the relationship between structure and function. According to different design purposes, the diversity of the studied sequences can be more precisely and intentionally controlled in the *in vitro* synthesized library. Moreover, the efficiency of obtaining nucleic acid or protein mutants can be significantly improved by means of the synthesized DNA library, which is helpful for downstream research.

Gene mutation library comprise alanine scanning library, degenerate mutation library, trimer library, site-directed saturation mutation library, random mutation library, *etc.*

*In vitro* molecular optimization can be significantly effectively used to generate improved or novel mutant proteins, identify regulatory sequences, and probe critical residues for structure and function. The construction of a synthetic library using the method of *in vitro* molecular optimization is a very effective way to systematically study the property, regulation and function of a protein.

By using the high-throughput semiconductor precise primer pool to synthesize all the primers theoretically required for the construction of the mutation library, according to different downstream expression systems, codon optimization can be performed and different mutation distribution rates at each mutation position can be set to fundamentally eliminate the problem of introducing unexpected stop codons. The library only contains the required mutants to save time and effort for subsequent screening.

However, current methods for constructing a gene mutation library have complicated steps and is time-consuming and labor-intensive. The existing method comprises further amplifying the mutated region synthesized by the chip by PCR, then amplifying the 2 unmutated regions before and after the mutated region by using a gene to be mutated as a template, and then assembling the 3 fragments into one fragment by assembly PCR, and finally amplifying the assembled full-length fragment by using the assembly product as a template and using the head and tail primers for the gene. In addition, purification must be performed after each step.

The present invention describes a method for constructing a gene mutation library, which can complete the construction of the gene mutation library through relatively simple steps and greatly saves the cost for constructing the gene mutation library.

Moreover, the gene mutation library generated by the current method for constructing a gene mutation library also has the problem that the mutation distribution does not meet the expectations.

The method for constructing a gene mutation library of the present invention can also realize that the ratio of sequences comprising various mutations in the obtained gene mutation library can be closer to the required ratio by simply optimizing the reaction conditions.

**Summary of the Invention**

A gene mutation library is very important for protein engineering and antibody drug engineering. A lot of time and effort for the subsequent construction and screening of gene mutation libraries can be saved by synthesis using high-throughput semiconductor precise primer pool. However, current methods for constructing a gene mutation library have complicated steps.

The present invention provides a new method for constructing a gene mutation library, which can complete the construction of the gene mutation library through relatively simple steps, solves the problem of complicated construction steps of the gene mutation library, and saves the cost for constructing the gene mutation library at the same time.

In a first aspect, the present invention provides a method for constructing a gene mutation library, which comprises:

(1) synthesizing a pool of oligonucleotide sequences comprising mutant nucleotide(s) at one or more mutation sites;
(2) performing PCR amplification on the oligonucleotides in the synthesized pool of the oligonucleotide sequences comprising the mutant nucleotides;
(3) performing PCR amplification by using the plasmid comprising a methylated site and a gene to be mutated or a part thereof as a template and using the oligonucleotides in the pool of the amplified oligonucleotide sequences obtained in step (2) as primers;
(4) using an endonuclease that recognizes and cleaves the methylated site to cleave the template plasmid present in the amplified system of step (3); and
(5) adding a forward primer and a reverse primer respectively corresponding to both ends of the gene to be mutated or the part thereof to the system obtained in step (4) and performing PCR amplification to obtain the gene mutation library comprising a plurality of genes containing the mutant nucleotide(s) or a part thereof.

In some embodiments, a purification step is not performed during step (1)-step (5) of the method for constructing a gene mutation library of the present invention.

In some embodiments, the method for constructing a gene mutation library of the present invention further comprises (6) recovering and purifying the amplified product of step (5) to obtain a final product. In some embodiments, step (6) comprises performing agarose gel electrophoresis on the amplified product of step (5), and then performing gel cutting, and recovering and purifying the amplified product, preferably using a DNA gel recovery kit for recovery and purification.

In some embodiments, the method for constructing a gene mutation library of the present invention further comprises (6) recovering and purifying the amplified product of step (5) to obtain a final product; and (7) sequencing the final product obtained in step (6) to verify sequence and/or detect gene mutation distribution. In some embodiments, the sequencing is Sanger sequencing and/or high-throughput sequencing.

In some embodiments, in step (1) of the method for constructing a gene mutation library of the present invention, the length of the oligonucleotide sequences in the pool of the pool synthesized oligonucleotide sequences is 60-170 bp, such as, 60, 70, 80, 90, 100, 110, 120, 130, 140, 150, 160 or 170 bp, preferably 80-90 bp, such as 80 or 90.

In some embodiments, in step (1) of the method for constructing a gene mutation library of the present invention, the oligonucleotides in the pool of the oligonucleotide sequences synthesized in step (1) comprise mutation nucleotides at 1 to 10 mutation sites, such as comprise mutation nucleotide(s) at 1, 2, 3, 4, 5, 6, 7, 8, 9 or 10 mutation sites. In some embodiments, in step (1) of the method for constructing a gene mutation library of the present invention, the oligonucleotides in the pool of the oligonucleotide sequences synthesized in step (1) comprise mutation nucleotides at 2 to 10 mutation sites. In some embodiments, the 2 to 10 mutation sites are either adjacent or non-adjacent.

In some embodiments, in the method for constructing a gene mutation library of the present invention, the amino acid sequence encoded by a gene comprising the mutant nucleotide(s) has at least one amino acid difference compared to the amino acid sequence encoded by the gene to be mutated. In some embodiments, the at least one amino acid difference is selected from: substitution, addition or deletion. Preferably, the at least one amino acid difference is substitution. In some embodiments, the substitution is to substitute the original amino acid with an amino acid having different properties from the original amino acid. Preferably, the substitution is to substitute the original amino acid with an amino acid having different properties from the original amino acid, wherein the properties are selected from: acidity and alkalinity, polarity, charge characteristic and side chain group. According to acidity and alkalinity of amino acids, amino acids are classified into acidic amino acids (lysine, arginine and histidine) and basic amino acids (aspartic acid and glutamic acid). According to the polarity of the side chain, amino acids are classified into non-polar amino acids (alanine, valine, leucine, isoleucine, proline, phenylalanine, tryptophan and methionine) and polar amino acids, wherein polar amino acids are classified into polar uncharged amino acids (glycine, serine, threonine, cysteine, tyrosine, aspartic acid and glutamine), polar positively charged amino acids (lysine, arginine and histidine), and polar negatively charged amino acids (aspartic acid and glutamic acid). According to the structure of the side chain group, amino acids are classified into aliphatic amino acids (alanine, valine, leucine, isoleucine, methionine, aspartic acid, glutamic acid, lysine, arginine, glycine, serine, threonine, asparagine, cysteine, and glutamine), aromatic amino acids (phenylalanine and tyrosine), heterocyclic amino acids (histidine and tryptophan), and heterocyclic imino acid (proline).

In some embodiments, in step (1) of the method for constructing a gene mutation library of the present invention, the mutant nucleotides of different mutant oligonucleotides in the pool of the synthesized oligonucleotide sequences can encode a mutant codon of an amino acid which is different from the amino acid encoded by the codon of corresponding site of the gene to be mutated, for example, if the site to be mutated in the gene to be mutated is alanine, then the nucleotide codon at this site of the different sequences in the pool of the synthesized oligonucleotide sequence can be a codon encoding different common amino acids, e.g., the codon encoding glycine, valine, leucine, isoleucine, proline, phenylalanine, tyrosine, tryptophan, serine, threonine, cysteine, methionine, asparagine, glutamine, aspartic acid, glutamic acid, lysine, arginine, and histidine. As another example, a specific mutant nucleotide codon in different sequences in a mutant gene library can encode an amino acid with properties different from the amino acid encoded by the original codon at the site to be mutated. For example, if the site to be mutated in the gene to be mutated is a codon encoding the non-polar amino acid alanine, then the corresponding mutation site in different oligonucleotide sequences in the pool of the oligonucleotide sequences can be a codon encoding a polar amino acid, e.g., a codon encoding tryptophan, serine, tyrosine, cysteine, methionine, asparagine, glutamine or threonine.

The purpose of step (2) of the method for constructing a gene mutation library of the present invention is to further increase the copy number of the oligonucleotides synthesized by the chip.

In some embodiments, in step (2) of the method for constructing a gene mutation library of the present invention, two primers respectively corresponding to both ends of the oligonucleotide sequences comprising the mutant nucleotide(s) at one or more mutation sites are used in the PCR amplification.

In step (2) of the method for constructing a gene mutation library of the present invention, the number of cycles of PCR amplification is minimized to avoid affecting the distribution of amino acids encoded by codons at the mutation sites. The frequency difference of the amino acids encoded by the codons at the mutation sites will be exponentially amplified with higher number of PCR cycles. Preferably, the number of cycles of PCR amplification is 10-30 cycles, and can be 10 to 28 cycles, 10 to 26 cycles, 10 to 28 cycles, 10 to 25 cycles, 10 to 22 cycles, 10 to 20 cycles, 10 to 18 cycles, 10 to

16 cycles, 12 to 30 cycles, 12 to 28 cycles, 12 to 26 cycles, 12 to 24 cycles, 12 to 22 cycles, 12 to 20 cycles, 12 to 18 cycles, 12 to 16 cycles, 12 to 14 cycles, 14 to 30 cycles, 14 to 28 cycles, 14 to 26 cycles, 14 to 24 cycles, 14 to 22 cycles, 14 to 20 cycles, 14 to 18 cycles, 14 to 16 cycles, 16 to 30 cycles, 16 to 28 cycles, 16 to 26 cycles, 16 to 24 cycles, 16 to 22 cycles, 16 to 20 cycles, and 16 to 18 cycles, more preferably 10 to 20 cycles, even more preferably 12 to 22 cycles, 14 to 16 cycles, e.g., 10, 11, 12, 13, 14, 15, 16, 17, 18, 19, 20, 21, 22, 23, 24, 25, 26, 27, 28, 29, 30 cycles, and most preferably 16 cycles.

In step (2) of the method for constructing a gene mutation library of the present invention, the DNA polymerase can be any DNA polymerase, preferably a high-fidelity DNA polymerase to reduce amplification errors, more preferably a high-fidelity Phusion DNA polymerase. The DNA polymerase can also be a high-fidelity DNA polymerase such as PrimeSTAR HS DNA Polymerase and Q5® High-Fidelity DNA Polymerase.

In step (3) of the method for constructing a gene mutation library of the present invention, performing PCR amplification by using the plasmid comprising a methylated site and a gene to be mutated or a part thereof as a template and using the oligonucleotides in the pool of the amplified oligonucleotide sequences obtained in step (2) as primers. The purpose of the step is to obtain the sequence of the gene comprising the mutant nucleotides at the mutation sites or a part thereof. There are no other primers in the reaction system, the primer for forward amplification lacks the corresponding forward primer, and the primer for backward amplification lacks the corresponding reverse primer. The plasmid contains the sequence of the full-length gene that needs to be mutated or a part thereof but not mutated. The oligonucleotide sequences in the pool of the amplified oligonucleotide sequences obtained in step (2) are double-stranded DNAs. In the denaturation stage of PCR, the double-stranded DNAs are dissociated into two single-stranded DNAs. The single-stranded DNAs are respectively used as the primer for the respective PCR reaction. In the respective PCR reaction, the forward linear amplification and the backward linear amplification are respectively performed by using the plasmid comprising the gene to be mutated or a part thereof as a template. Linear amplification is the linear amplification of DNA molecules. The number of finally obtained DNA molecules is much smaller than that of exponential amplification and the number of primers required is also much smaller than exponential amplification. Therefore, in the linear amplification of step (3), it is necessary to strictly control the number of primers and the number of templates in the system, and minimize the number of cycles of PCR reaction as far as possible.

In some embodiments, in step (3) of the method for constructing a gene mutation library of the present invention, it is necessary to strictly control the amount of the primers (*i.e.,* the oligonucleotides in the pool of the amplified oligonucleotide sequence obtained in step (2)) and the amount of the template (*i.e.,* the plasmid comprising a methylated site and a gene to be mutated or a part thereof) in the system. The inventors found that when the molar ratio of primers to templates is 20 : 1, the amplification effect is the best. In some embodiments, the molar ratio of primers to templates is 15 : 1 to 100 : 1, preferably 15 : 1 to 90 : 1, 15 : 1 to 80 : 1, 15 : 1 to 70 : 1, 15 : 1 to 60 : 1, 15 : 1 to 50 : 1, 15 : 1 to 40 : 1, 15 : 1 to 30 : 1, 15 : 1 to 28: 1, 15 : 1 to 26: 1, 15 : 1 to 24 : 1, 15 : 1 to 22 : 1, 15 : 1 to 20 : 1, and 16 : 1 to 100 : 1; preferably 16 : 1 to 90 : 1, 16 : 1 to 80 : 1, 16 : 1 to 70 : 1, 16 : 1 to 60 : 1, 16 : 1 to 50 : 1, 16 : 1 to 40 : 1, 16 : 1 to 30 : 1, 16 : 1 to 28 : 1, 16 : 1 to 26 : 1, 16 : 1 to 24 : 1, 16 : 1 to 22 : 1, and 16 : 1 to 20 : 1; preferably 17 : 1 to 90 : 1, 17 : 1 to 80 : 1, 17 : 1 to 70 : 1, 17 : 1 to 60 : 1, 17 : 1 to 50 : 1, 17 : 1 to 40 : 1, 17 : 1 to 30 : 1, 17 : 1 to 28 : 1, 17 : 1 to 26 : 1, 17 : 1 to 24 : 1, 17 : 1 to 22 : 1, and 17 : 1 to 20 : 1; preferably 18 : 1 to 90 : 1, 18 : 1 to 80 : 1, 18 : 1 to 70 : 1, 18 : 1 to 60 : 1, 18 : 1 to 50 : 1, 18 : 1 to 40 : 1, 18 : 1 to 30 : 1, 18 : 1 to 28 : 1, 18 : 1 to 26 : 1, 18 : 1 to 24 : 1, 18 : 1 to 22 : 1, and 18 : 1 to 20 : 1; preferably 19 : 1 to 90 : 1, 19 : 1 to 80 : 1, 19 : 1 to 70 : 1, 19 : 1 to 60 : 1, 19 : 1 to 50 : 1, 19 : 1 to 40 : 1, 19 : 1 to 30 : 1, 19 : 1 to 28 : 1, 19 : 1 to 26 : 1, 19 : 1 to 24 : 1, 19 : 1 to 22 : 1, and 19 : 1 to 20 : 1; preferably 20 : 1 to 90 : 1, 20 : 1 to 80 : 1, 20 : 1 to 70 : 1, 20 : 1 to 60 : 1, 20 : 1 to 50 : 1, 20 : 1 to 40 : 1, 20 : 1 to 30 : 1, 20 : 1 to 28 : 1, 20 : 1 to 26 : 1, 20 : 1 to 24 : 1, and 20 : 1 to 22 : 1, and most preferably 20 : 1.

In step (3) of the method for constructing a gene mutation library of the present invention, the number of cycles of PCR amplification is minimized to avoid affecting the distribution of amino acids encoded by nucleotides at the mutation sites. The frequency difference of the amino acids encoded by the mutation sites will be exponentially amplified with higher number of PCR cycles. Preferably, the number of cycles of PCR amplification is 10-30 cycles, and can be 10 to 28 cycles, 10 to 26 cycles, 10 to 28 cycles, 10 to 25 cycles, 10 to 22 cycles, 10 to 20 cycles, 10 to 18 cycles, 10 to 16 cycles, 12 to 30 cycles, 12 to 28 cycles, 12 to 26 cycles, 12 to 24 cycles, 12 to 22 cycles, 12 to 20 cycles, 12 to 18 cycles, 12 to 16 cycles, 12 to 14 cycles, 14 to 30 cycles, 14 to 28 cycles, 14 to 26 cycles, 14 to 24 cycles, 14 to 22 cycles, 14 to 20 cycles, 14 to 18 cycles, 14 to 16 cycles, 16 to 30 cycles, 16 to 28 cycles, 16 to 26 cycles, 16 to 24 cycles, 16 to 22 cycles, 16 to 20 cycles, and 16 to 18 cycles, more preferably 10 to 20 cycles, even more preferably 12 to 22 cycles, 14 to 16 cycles, e.g., 10, 11, 12, 13, 14, 15, 16, 17, 18, 19, 20, 21, 22, 23, 24, 25, 26, 27, 28, 29, 30 cycles, and most preferably 15 cycles.

In step (3) of the method for constructing a gene mutation library of the present inventio, according to the length of the gene to be amplified or a part thereof, the two PCR reactions in which the two strands of the oligonucleotides in the pool of the amplified oligonucleotide sequences obtained in step (2) are used as primers can respectively extend to the head and tail ends of the gene or a part thereof by controlling the extension time of the PCR reaction.

In step (4) of the method for constructing a gene mutation library of the present invention, an endonuclease that recognizes and cleaves the methylated site is used to cleave the template plasmid present in the amplified system of step (3) and the purpose is to avoid amplifying the plasmid that does not contain the mutation as the template for the next step to obtain a final gene product that does not contain the mutation. The plasmids extracted from bacteria all have methylated sites and the products obtained by PCR amplification in step (3) do not contain methylated site, therefore an endonuclease that recognizes and cleaves the methylated sites can selectively cleave the plasmids into small fragments and thus cannot be used as the template for the next PCR reaction.

In some embodiments, in the method for constructing a gene mutation library of the present invention, the plasmid is extracted from bacteria. In some embodiments, the plasmid is extracted from *E. coli*. In some embodiments, the plasmid is extracted from *E. coli* TOP10. Bacteria generally have methylases to methylate the DNA of the bacteria. For example, Dam methylase is present in *E. coli* TOP10. Dam methylase recognizes and methylates adenines in GATC motif.

In some embodiments, in step (4) of the method for constructing a gene mutation library of the present invention, endonucleases that recognize and cleave methylated sites are well known in the art. For example, the endonucleases that recognize and cleave methylated sites are *Dpn*I, *Msp*JI, *Fsp*EI etc. Preferably, the endonucleases that recognize and cleave methylated sites are *Dpn*I. Restriction endonuclease *Dpn*I can effectively recognize and cut adenine methylated G$^m$ATC, but cannot cut unmethylated GATC sequence. Plasmids extracted from Dam+ bacteria all contain G$^m$ATC, which can act as substrate for Dpn I. Preferably, the plasmid that can be digested by *Dpn*I is extracted from *E. coli* TOP10 strain.

In some embodiments, in the method for constructing a gene mutation library of the present invention, the plasmid having methylated sites is pMB1, pBR322, pcDNA3.1 or pUC57. In some embodiments, the plasmid having methylated sites is pMB1.

In some embodiments, in step (5) of the method for constructing a gene mutation library of the present invention, a forward primer and a reverse primer respectively corresponding to both ends of the gene to be mutated or a part thereof are added to the system obtained in step (4) and PCR amplification is performed to obtain a gene mutation library comprising a plurality of genes containing mutant nucleotide(s) or a part thereof. The template of the PCR reaction is two PCR amplification products obtained in step (3) with different orientations linked together by complementary base pairing.

In step (5) of the method for constructing a gene mutation library of the present invention, the number of cycles of PCR amplification is minimized to avoid affecting the distribution of amino acids encoded by codons at the mutation sites. The frequency difference of the amino acids at the mutation sites will be exponentially amplified with higher number of PCR cycles. Preferably, the number of cycles of PCR amplification is 10-30 cycles, and can be 10 to 28 cycles, 10 to 26 cycles, 10 to 28 cycles, 10 to 25 cycles, 10 to 22 cycles, 10 to 20 cycles, 10 to 18 cycles, 10 to 16 cycles, 12 to 30 cycles, 12 to 28 cycles, 12 to 26 cycles, 12 to 24 cycles, 12 to 22 cycles, 12 to 20 cycles, 12 to

18 cycles, 12 to 16 cycles, 12 to 14 cycles, 14 to 30 cycles, 14 to 28 cycles, 14 to 26 cycles, 14 to 24 cycles, 14 to 22 cycles, 14 to 20 cycles, 14 to 18 cycles, 14 to 16 cycles, 16 to 30 cycles, 16 to 28 cycles, 16 to 26 cycles, 16 to 24 cycles, 16 to 22 cycles, 16 to 20 cycles, and 16 to 18 cycles, more preferably 10 to 20 cycles, even more preferably 12 to 22 cycles, 14 to 16 cycles, e.g., 10, 11, 12, 13, 14, 15, 16, 17, 18, 19, 20, 21, 22, 23, 24, 25, 26, 27, 28, 29, 30 cycles, and most preferably 16 cycles.

After step (5) of the method for constructing a gene mutation library of the present invention, preferably, the step further comprises subjecting the amplified product to agarose gel electrophoresis, recovery and/or purification to obtain a final product.

Further, the method for constructing a gene mutation library of the present invention also comprises step (6): sequencing the final product obtained in step (5) to verify whether the sequence is correct. Preferably, the sequencing is Sanger sequencing. The step comprises performing the blunt-end ligation of the final product to ligate to linearized Puc57-EV, and sequencing the final product by using M13F primer. Preferably, the sequencing is high-throughput sequencing or next generation sequencing technology (NGS for short), and is used to verify whether the sequence is correct, and the sequencing can also be used to detect the proportion of various nucleotide mutations at each mutation site respectively, so as to verify that the proportion matches the desired proportion.

In a second aspect, the present invention provides a gene mutation library, which is constructed by the method for constructing a gene mutation library of the present invention.

In a third aspect, the present invention provides a kit for the method for constructing a gene mutation library above, the kit comprises a pool of oligonucleotide sequences comprising mutant nucleotide(s) at one or more mutation sites, a plasmid comprising a methylated site and a gene comprising nucleotides to be mutated or a part thereof, and an endonuclease that recognizes and cleaves the methylated site. In some embodiments, the kit also comprises DNA polymerase.

In a fourth aspect, the present invention provides a method for analyzing the relationship between an amino acid mutation in a protein and the properties, regulation and/or function of the protein, comprising the following steps:

(1) using the method of the present invention to construct a gene mutation library of a gene encoding the protein;
(2) comparing the properties, regulation and/or function of the protein encoded by a mutant gene in the gene mutation library with the properties, regulation and/or function of the unmutated protein; and
(3) analyzing the relationship between the amino acid at the mutation position and the properties, regulation and/or function of the protein.

Specifically, the present invention relates to the following items:

1. A method for constructing a gene mutation library, comprising:

(1) synthesizing a pool of oligonucleotide sequences comprising mutant nucleotide(s) at one or more mutation sites;
(2) performing PCR amplification on the oligonucleotides in the synthesized pool of the oligonucleotide sequences comprising the mutant nucleotides;
(3) performing PCR amplification by using the plasmid comprising a methylated site and a gene to be mutated or a part thereof as a template and using the oligonucleotides in the pool of the amplified oligonucleotide sequences obtained in step (2) as primers;
(4) using an endonuclease that recognizes and cleaves the methylated site to cleave the template plasmid present in the amplified system of step (3); and
(5) adding a forward primer and a reverse primer respectively corresponding to both ends of the gene with nucleotides to be mutated or the part thereof to the system obtained in step (4) and performing PCR amplification to obtain the gene mutation library comprising a plurality of genes containing the mutation or a part thereof.

2. The method according to item 1, wherein purification is not performed during step (1)-step (5).
3. The method according to item 1, further comprising
(6) recovering and purifying the amplified product of step (5) to obtain a final product.
4. The method according to item 3, further comprising
(7) sequencing the final product obtained in step (6) to verify sequence and/or detect gene mutation distribution.
5. The method according to item 4, wherein the sequencing is Sanger sequencing and/or high-throughput sequencing.
6. The method according to any one of items 1-5, wherein the length of the oligonucleotide sequences in the pool of the oligonucleotide sequences synthesized in step (1) is 60-170 bp, preferably 80-90 bp.
7. The method according to any one of items 1-6, wherein the pool of the oligonucleotide sequences synthesized in step (1) comprises mutant nucleotide(s) at 1 to 10 mutation sites.
8. The method according to any one of items 1-7, wherein the mutation sites are either adjacent or non-adjacent.
9. The method according to any one of items 1-8, wherein the amino acid sequence encoded by a gene comprising the mutant nucleotide(s) has at least one amino acid difference compared to the amino acid sequence encoded by the gene to be mutated.
10. The method according to item 9, wherein the at least one amino acid difference is selected from: substitution, addition or deletion.

11. The method according to item 10, wherein the at least one amino acid difference is substitution.

12. The method according to item 11, wherein the substitution is to substitute the original amino acid with an amino acid having different properties from the original amino acid.

13. The method according to item 12, wherein the properties are selected from: acidity and alkalinity, polarity, charge characteristic and side chain group.

14. The method according to any one of items 1-13, wherein two primers respectively corresponding to both ends of the oligonucleotide sequences comprising the mutant nucleotide(s) at one or more mutation sites are used in the PCR amplification in step (2).

15. The method according to any one of items 1-14, wherein the number of cycles of the PCR amplification in step (2) is 10-25 cycles, preferably 12-22 cycles, 14-20 cycles, 15-18 cycles or 15-16 cycles, and most preferably 16 cycles.

16. The method according to any one of items 1-15, wherein the DNA polymerase used in the PCR amplification in step (2) is a high-fidelity DNA polymerase.

17. The method according to any one of items 1-16, wherein the molar ratio of the primers to the template in step (3) is 15 : 1 to 50 : 1, preferably 16 : 1 to 40 : 1, 17 : 1 to 30 : 1, 18 : 1 to 25 : 1, or 19 : 1 to 22 : 1, more preferably 19 : 1 to 21 : 1, and most preferably 20 : 1.

18. The method according to any one of items 1-17, wherein the number of cycles of the PCR amplification in step (3) is 10-20 cycles, preferably 12-18 cycles, 14-16 cycles, and most preferably 15 cycles.

19. The method according to any one of items 1-18, wherein the DNA polymerase used in the PCR amplification in step (3) is a high-fidelity DNA polymerase, preferably a high-fidelity Phusion DNA polymerase.

20. The method according to any one of items 1-19, wherein the plasmid is extracted from bacteria that methylate the plasmid, preferably from *E. coli,* more preferably from *E. coli* TOP10 strain.

21. The method according to any one of items 1-20, wherein the endonuclease in step (4) is *Dpn*I, *Msp*JI or *Fsp*EI.

22. A gene mutation library constructed using the method according to any one of items 1-21.

23. A kit for the method for constructing a gene mutation library according to any one of items 1-21, comprising a pool of oligonucleotide sequences comprising mutant nucleotide(s) at one or more mutation sites, a plasmid comprising a methylated site and a gene to be mutated or a part thereof, and an endonuclease that recognizes and cleaves the methylated site.

24. The kit according to item 23, wherein the kit comprises a DNA polymerase.

25. A method for analyzing the relationship between an amino acid mutation in a protein and the properties, regulation and/or function of the protein, comprising the following steps:

(1) using the method according to any one of items 1-21 to construct a gene mutation library of a gene encoding the protein;

(2) comparing the properties, regulation and/or function of the protein encoded by a mutant gene in the gene mutation library with the properties, regulation and/or function of the unmutated protein; and

(3) analyzing the relationship between the amino acid at the mutation position and the properties, regulation and/or function of the protein.

**Brief Description of the Drawings**

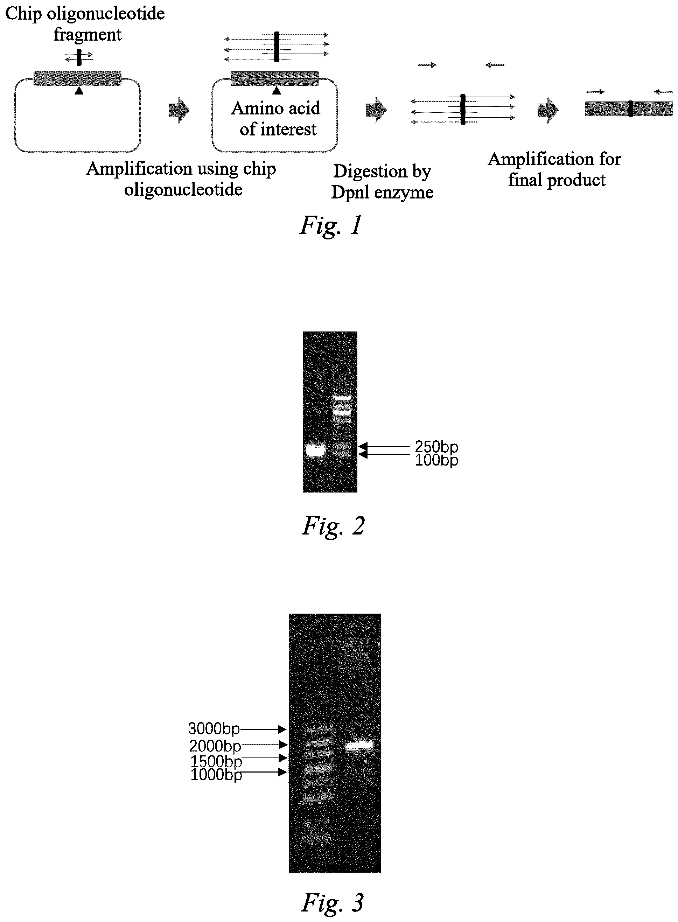

Fig. 1 is a schematic flow diagram of the method for constructing a gene mutation library of the present invention.

Fig. 2 is an electrophoretogram of the product obtained by performing PCR amplification on the oligonucleotides in the pool of the synthesized oligonucleotide sequences comprising the mutant nucleotides. The right lane is DL3000 marker, and the left lane is the amplification product using Chip-F and Chip-R as primers and using the pool of the synthesized oligonucleotide sequences comprising the mutant nucleotides as the template.

Fig. 3 is an electropherogram of the final product obtained in Example 5. The left lane is DL3000 marker, and the right lane is the final product obtained by agarose gel electrophoresis, recovery and purification of the product obtained by performing amplification using Chip-F and Chip-R as primers and using the single-stranded DNAs linked together by complementary regions as the template. The single-stranded DNAs are the sense and antisense PCR amplification products obtained by performing amplification respectively using the two strands of the synthesized oligonucleotides in Example 3 as primers.

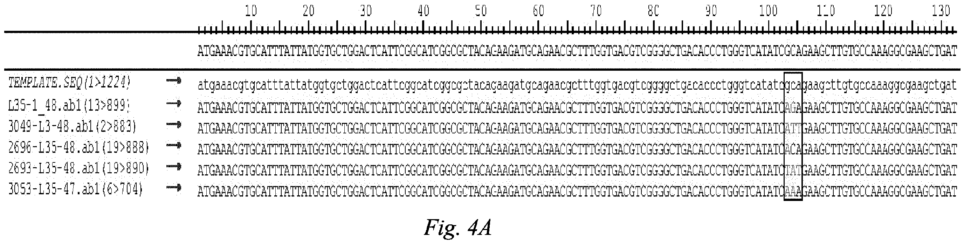

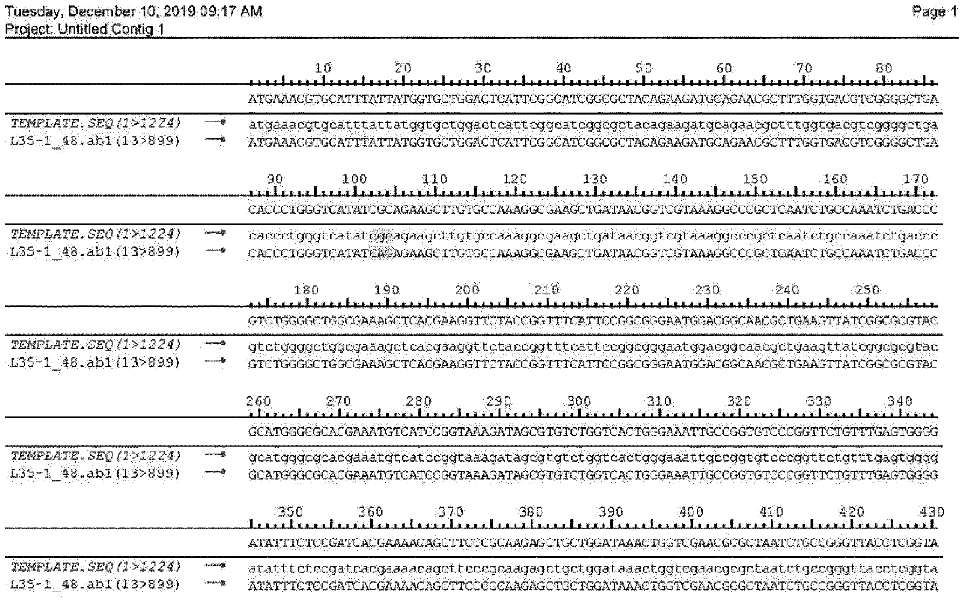

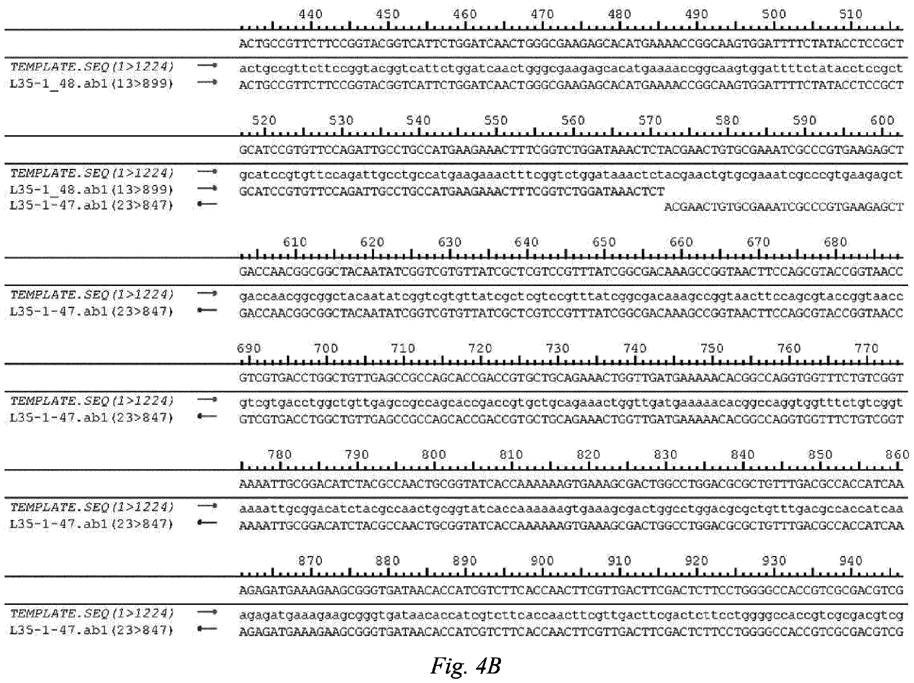

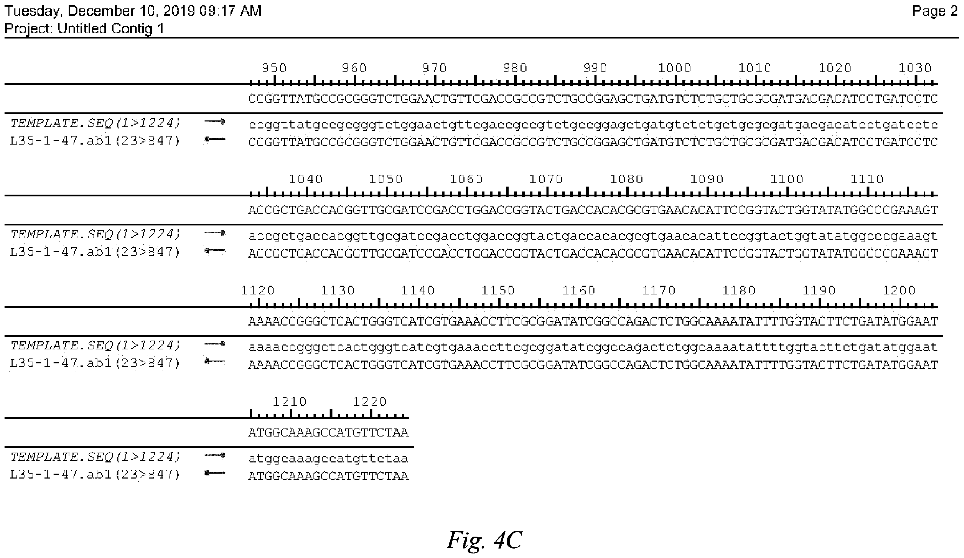

Figs. 4A-4C are the results of Sanger sequencing of the final product. At the target mutation sites, *i.e.,* at the codon positions of amino acid at position 35, i.e., at the nucleotides at positions 103-105, a mutation occurred.

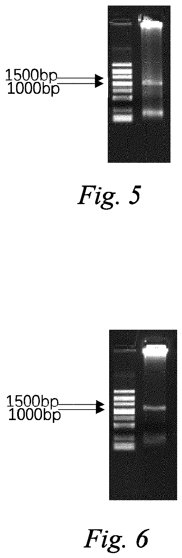

Fig. 5 is an electropherogram of the final product in the step of linear amplification using the synthesized oligonucleotides as primers and using the molar ratio of templates to primers of 1 : 254, and the cycle number of PCR reaction of 25. The left lane is DL3000 marker, and the right lane is the final product obtained by performing amplification using Chip-F and Chip-R as primers and using the single-stranded DNAs linked together by complementary regions as the template. The single-stranded DNAs are the sense and antisense PCR amplification products obtained by performing amplification respectively using the two strands of the synthesized oligonucleotides in Example 7 as primers.

Fig. 6 tests the situation when the molar ratio of templates to primers is 1 : 254 and the cycle number of PCR reaction is 15 in the step of linear amplification with the synthesized oligonucleotides as primers. The left lane is DL3000 marker, and the right lane is the final product obtained by performing amplification using Chip-F and Chip-R as primers and using the single-stranded DNAs linked together by complementary regions as the template. The single-stranded DNAs are the sense and antisense PCR amplification products obtained by performing amplification respectively using the two strands of the synthesized oligonucleotides in Example 7 as primers.

## Detailed Description of Embodiments

As used in the present description and appended claims, the singular forms "a," "an," and "the" include plural referents, unless the context clearly dictates otherwise. Therefore, for example, reference to "a molecule" optionally includes a combination of two or more of such molecules, and the like.

As used herein, the term "about" refers to a conventional error range of the corresponding numerical value easily known by those skilled in the art. Reference herein to "about" a certain value or a parameter includes (and describes) the embodiment involving the value or parameter itself.

It is understood that the aspects and embodiments of the present invention described herein include aspects and embodiments involving "comprise", "consist of', and "essentially consist of".

The term "oligonucleotide" refers to a general term for short-chain nucleotides. In this context, the oligonucleotide can be made by polymerizing 2-300 nucleotides via phosphodiester bonds.

The term "pool of oligonucleotide sequences" refers to a mixture consisting of a variety of different oligonucleotide sequences.

The term "gene mutation library" refers to a mixture consisting of a variety of different DNA variant sequences. A Gene mutation library is a product of the combination of gene synthesis, gene mutation and directed evolution study. The gene mutation library has been increasingly used in research fields, such as high-throughput drug target screening, directed evolution by protein engineering, synthesizing diversity antibody libraries to screen variant antibodies with high-affinity and specificity, *etc.*

The term "PCR amplification" refers to the polymerase chain reaction, which is a molecular biology technique for amplifying a specific DNA fragment. It can be regarded as a special DNA replication *in vitro.* The biggest feature of PCR is that it can greatly increase the trace amount of DNA. Specifically, the basic principle of PCR technology is similar to the natural replication process of DNA, and its specificity relies on oligonucleotide primers complementary to both ends of the target sequence. PCR is composed of three basic reaction steps: denaturation, annealing and extension, and comprises: (1) denaturation of a template DNA: heating the template DNA to above 90°C for a certain period of time, and then dissociating the double-stranded template DNA or the double-stranded DNA formed by PCR amplification to become single strands, so that the single strands can be bound with primers to prepare for the next round of reaction; (2) annealing (renaturation) of the template DNA and primers: after the template DNA is denatured into a single strand by heating, lowering the temperature to a lower temperature, wherein the primers are paired and bound with the complementary sequence of the single-stranded template DNA; (3) extension of primers: synthesizing a new semi-reserved replication strand complementary to the template DNA strand from the DNA template-primer conjugate, under the action of DNA polymerase (such as TaqDNA polymerase) at about 70°C, using dNTPs as the reaction raw material, using the target sequence as a template, and according to the principles of complementary base pairing and semi-reserved replication. More "semi-retained replicated strands" can be obtained by repeating the cycle of the three processes of denaturation-annealing-extension.

In step (5) of the method for constructing a gene mutation library of the present application, a forward primer and a reverse primer respectively corresponding to both ends of the gene to be mutated or a part thereof are added to the system obtained in step (4) and PCR amplification is performed to obtain a gene mutation library comprising a plurality of genes containing mutant nucleotide(s) or a part thereof. In this step, fusion PCR is performed and comprises (1) denaturation of template DNA: heating the template DNA to above 90°C for a certain period of time to keep the template DNA in the original single-stranded state, wherein the template DNA is a product obtained by performing PCR amplification using the plasmid containing the gene to be mutated in step (3) as a template and using the oligonucleotides in the pool of the oligonucleotides amplified in step (2) as primers, and is complementary at positions corresponding to the oligonucleotide sequences in the pool of the oligonucleotides synthesized in step (1); (2) annealing: annealing the template DNAs, wherein the complementary sequence of the single-stranded template DNA is paired and bound; (3) extension: using DNA polymerase to extend the DNA strand from the 3' end to form a complete double-stranded DNA by using the complementary strand as a template. Then, the subsequent PCR reaction is the same as ordinary PCR, a gene mutation library comprising a plurality of genes containing mutant nucleotide(s) or a part thereof is obtained by the denaturation/annealing/extension three-step amplification, using the formed complete double-stranded DNA as a template and using the added forward primer and reverse primer corresponding to the two ends of the gene to be mutated or a part thereof as primers.

The term "PCR primer" refers to a synthesized macromolecule with a specific nucleotide sequence, that is paired and bound with the complementary sequence of the single-stranded template DNA during the annealing (renaturation) stage of the polymerase chain reaction and is linked with the nucleotides in the reaction system via covalent bonds under the action of DNA polymerase during extension stage.

Usually, one primer in the primer pair is complementary to one DNA template strand at one end of the target region, and the other primer in the primer pair is complementary to the other DNA template strand at the other end of the target region. The function of the primer is to serve as the starting point of nucleotide polymerization, and nucleic acid polymerases can respectively start to synthesize new nucleic acid strands from the 3' ends of the two primers. The synthesized double-stranded DNA is re-melted during the DNA denaturation stage of the next cycle and can be used as a template of this cycle for amplification. Theoretically, this amplification method produces $m*2^n$ double-stranded DNAs, where m is the number of template strands initially added, and n is the number of cycles. Therefore, PCR amplification using this primer pair is also called exponential amplification.

The primer pair herein can also be two complementary oligonucleotides, wherein the two oligonucleotides are melted during the denaturation stage, complementary to respectively corresponding templates thereof during the annealing stage, and extended and amplified in the forward and backward directions respectively. The double-stranded DNA obtained after amplification is a complex of the template strand initially added to the system and the product strand obtained by extension initiated from the primer. In this case, the product strand cannot be used as the template for the next amplification reaction. Only the template strand initially added to the system can be used as a template for each PCR cycle. Therefore, theoretically, this amplification method respectively produces $m*n$ single-stranded DNAs in each direction after n cycles, where m is the number of template strands added initially, and n is the number of cycles. Therefore, PCR amplification using such primer pairs is a linear amplification.

The term "DNA polymerase" is an enzyme that replicates DNA from the 5' end to the 3' end using DNA as a template for replication. The main activity of DNA polymerase is to catalyze the synthesis of DNA in the presence of templates, primers, dNTPs, *etc.*

The term "high-fidelity DNA polymerase" refers to a DNA polymerase with a low incorporation error rate and proofreading activity to ensure faithful replication of the DNA of interest.

The term "gene to be mutated" herein refers to a gene that needs to be specifically mutated to construct a gene mutation library, and the sequence of the gene to be mutated is an original sequence that does not contain a mutant nucleotide.

The term "plasmid" refers to a DNA molecule other than chromosomal DNA (or nucleoid) in organisms such as bacteria. Plasmid exists in the cytoplasm and has the ability to autonomously replicate, allowing same to exist in daughter cells, maintain a constant copy number, and express all carried genetic information. In general, plasmids extracted from bacteria have methylated sites.

The term "DNA methylation" is a form of chemical modification of DNA that can alter genetic behavior without altering the DNA sequence. In a broad sense, DNA methylation refers to a chemical modification process in which a specific base on the DNA sequence obtains a methyl group via covalent binding under the catalysis of DNA methyltransferase (DNMT) and using S-adenosyl methionine (SAM) as a methyl donor. This DNA methylation modification can occur at positions, such as the C-5 position of cytosine, the N-6 position of adenine and the N-7 position of guanine. DNA methylation, as a stable modification state, can be inherited to the new daughter DNA along with the DNA replication process under the action of DNA methyltransferase, which is an important epigenetic mechanism.

Common K12 *E. coli* strains in laboratory, such as TOP10 comprise 3 methylases that recognize different DNA sequences: Dam methylase, which adds a methyl to the A of the GATC motif of DNA; Dcm methylase, which adds a methyl to the second C of the CCWGG motif of DNA; and EcoKI methylase, which adds a methyl to the A of AACNNNNNNGTGC motif or GCACNNNNNNGTT motif of DNA.

The term "restriction endonuclease" refers to a class of enzymes that can recognize and attach to a specific deoxynucleotide sequence and cleave the phosphodiester bond between two deoxyribonucleotides at a specific location in each strand, and is referred to as "endonuclease".

The term "restriction site" refers to a specific sequence on a DNA strand, wherein the restriction endonuclease can specifically recognize the sequence and cleave the DNA sequence here into two fragments in a specific manner.

The term "endonuclease that recognizes and cleaves a methylated site" refers to a class of enzymes that can recognize and attach to a specific deoxynucleotide sequence comprising a methylated deoxynucleotide and cleave the phosphodiester bond between two deoxyribonucleotides at a specific location in each strand. For example, the endonuclease that recognizes and cleaves a methylated site can be *Dpn*I enzyme, *Fsp*EI enzyme and *Msp*JI.

*Dpn*I enzyme recognizes and cleaves the methylated motif C$^m$ATC in plasmids extracted from bacteria containing Dam methylase. However, unmethylated PCR products are not cleaved because PCR system does not contain any methylase. Particularly, "$^m$A" in the motif "G$^m$ATC" represents a methylated adenine nucleotide.

*Fsp*EI enzyme is a modification-dependent endonuclease that recognizes C$^m$C site and creates a double-stranded DNA break at the 3' end $N_{12}/N_{16}$ of modified cytosine C, *i.e.,*

5´...C$^m$C (N)$_{12}$ ▼...3´
3´...G G (N)$_{16}$ ▲...5´

. Therefore, *Fsp*EI enzyme recognizes and cleaves the methylated motif C$^m$CWGG in plasmids extracted from bacteria containing Dcm methylase. However, unmethylated PCR products are not cleaved because PCR system does not contain any methylase. Particularly, "$^m$C" in the motifs "C$^m$C" and "C$^m$CWGG" represents a methylated cytosine nucleotide.

*Msp*JI enzyme is a modification-dependent endonuclease that recognizes $^m$CNNR site and creates a double-stranded DNA break at the 3' end $N_9/N_{13}$ of modified cytosine, *i.e.,*

5´...$^m$C N N R (N)$_9$ ▼...3´
3´... G N N Y (N)$_{13}$ ▲...5´

, wherein R = A or G; and Y = C or T. Particularly, "$^m$C" in the motif "$^m$CNNR" represents a methylated cytosine nucleotide.

Sanger sequencing is a method, which comprises starting at a fixed point and randomly ending at a specific base of the nucleotide, and fluorescently labelling each base to generate a series of four groups of nucleosides with different lengths ending with A, T, C, and G, and then detecting by electrophoresis on a urea-denatured PAGE gel to obtain visible DNA base sequences.

High-throughput sequencing, also known as "Next-generation" sequencing technology (abbreviated as NGS), is relative to the traditional Sanger sequencing method. NGS is characterized by the ability to sequence hundreds of thousands to millions of DNA molecules in parallel and generally shorter reads. At present, the main platforms for high-throughput sequencing are represented by Roche 454 sequencer (Roch GS FLX sequencer), Illumina Solexa genome analyzer (Illumina Genome Analyzer) and ABI SOLiD sequencer. The common features thereof are (1) the DNA of interest is cut into small fragments; (2) the single small fragment DNA molecule binds to the surface of a solid phase; (3) the single molecule is amplified independently; (4) only one base (A, C, T, and G) is copied at one time and the signal is detected; and (5) high-resolution imaging system.

The term "Sequencing Depth" refers to the ratio of the total number of bases (bp) obtained by sequencing to the size of the genome, which is one of the indicators for evaluating the amount of sequencing. There is a positive correlation between sequencing depth and genome coverage, and the error rate or false positive results brought by sequencing will decrease with the increase of sequencing depth. In this context, the sequencing depth is used in the sequencing results obtained by high-throughput sequencing of gene mutation library. In this context, the sequencing depth refers specifically to the sequencing depth of each gene mutant in high-throughput sequencing results.

The term "average sequencing depth" herein refers to the average sequencing depth of each gene mutant in the sequencing results of the gene mutation library.

The term "sequencing depth ranked 10%" refers to the sequencing depth corresponding to the gene mutant ranked 10%, wherein all the gene mutants obtained in the sequencing results of the gene mutation library are ranked according to the corresponding sequencing depth thereof from small to large.

The term "sequencing depth ranked 90%" refers to the sequencing depth corresponding to the gene mutant ranked 90%, wherein all the gene mutants obtained in the sequencing results of the gene mutation library are ranked according to the corresponding sequencing depth thereof from small to large.

The term "deviation degree" refers to the sequencing depth ranked 90%/sequencing depth ranked 10%, which is used to characterize whether the sequencing depths of all the gene mutants obtained by high-throughput sequencing of the gene mutation library is uniform, and indicates whether the copy numbers of different mutant sequences comprising different mutations at a certain mutation site are uniform in the gene mutation library. 1 is the optimal deviation degree, which denotes that in the gene mutation library, the copy numbers of different mutation sequences comprising different mutations at a certain mutation site are same. The closer the deviation degree is to 1, the closer the copy numbers of different mutant sequences comprising different mutations at a certain mutation site are.

The term "gene mutation distribution" herein refers to the overall distribution of mutations at a particular position for all sequences in the finally obtained gene mutation library.

**Description Of Sequences**

| SEQ ID NO | Description Of Sequences |
|---|---|
| 1 | Amino acid sequence of the protein encoded by the original gene for which the gene mutation library needs to be constructed |
| 2 | Nucleotide sequence of the original gene for which the gene mutation library needs to be constructed |
| 3 | Oligonucleotide comprising mutations synthesized by Chip |
| 4 | Oligonucleotide comprising mutations synthesized by Chip |
| 5 | Oligonucleotide comprising mutations synthesized by Chip |
| 6 | Oligonucleotide comprising mutations synthesized by Chip |
| 7 | Oligonucleotide comprising mutations synthesized by Chip |
| 8 | Oligonucleotide comprising mutations synthesized by Chip |
| 9 | Oligonucleotide comprising mutations synthesized by Chip |
| 10 | Oligonucleotide comprising mutations synthesized by Chip |
| 11 | Oligonucleotide comprising mutations synthesized by Chip |
| 12 | Oligonucleotide comprising mutations synthesized by Chip |
| 13 | Oligonucleotide comprising mutations synthesized by Chip |
| 14 | Oligonucleotide comprising mutations synthesized by Chip |
| 15 | Oligonucleotide comprising mutations synthesized by Chip |
| 16 | Oligonucleotide comprising mutations synthesized by Chip |
| 17 | Oligonucleotide comprising mutations synthesized by Chip |
| 18 | Oligonucleotide comprising mutations synthesized by Chip |
| 19 | Oligonucleotide comprising mutations synthesized by Chip |
| 20 | Oligonucleotide comprising mutations synthesized by Chip |
| 21 | Oligonucleotide comprising mutations synthesized by Chip |
| 22 | Forward primer for PCR amplification of the synthesized oligonucleotide comprising mutations |
| 23 | Reverse primer for PCR amplification of the synthesized oligonucleotide comprising mutations |
| 24 | Forward primer for PCR amplification of the full-length gene comprising mutations |
| 25 | Reverse primer for PCR amplification of the full-length gene comprising mutations |

**Specific Examples**

**Example 1. Synthesis of an oligonucleotide**

The amino acid sequence for which the library needs to be constructed was as follows: MKRAFIMVLDSFGIGATEDAERFGDVGADTLGHI**A**EACAKGEADNGRKG PLNLPNLTRLGLAKAHEGSTGFIPAGM-DGNAEVIGAYAWAHEMSSGKDS VSGHWEIAGVPVLFEWGYFSDHENSFPQELLDKLVERANLPGYLGNCRS SGTVILDQLGEEHMKTGKWIFYTSAASVFQIACHEETFGLDKLYELCEIA REELTNGGYNIGRVIARPFIGDKAGN-FQRTGNRRDLAVEPPAPTVLQKLV DEKHGQVVSVGKIADIYANCGITKKVKATGLDALFDATIKEMKEAGDNT IVFTNFVDFDSSWGHRRDVAGYAAGLELFDRRLPELMSLLRDDDILILTA DHGCDPTWTGTDHTREHIPVLVYGPKVK-PGSLGHRETFADIGQTLAKYF GTSDMEYGKAMF* (SEQ ID NO: 1), a total of 407 amino acids, wherein the 35th amino acid is the amino acid which needs to be mutated, that is, the alanine (Ala) is needed to be mutated into another 19 common amino acids, i.e., glycine (Gly), valine (Val), leucine (Leu), isoleucine (Ile), phenylalanine (Phe), proline (Pro), tryptophan (Trp), serine (Ser), tyrosine (Try), cysteine (Cys), methionine (Met), asparagine (Asn), glutamine (Gln), threonine (Thr), aspartic acid (Asp), glutamic acid (Glu), lysine (Lys), arginine (Arg) and histidine (His).

The nucleotide sequence of the full-length gene which needs to be mutated is as follows (1224 bp in total):

atgaaacgtgcatttattatggtgctggactcattcggcatcggcgctac<u>agaagatgcagaacgctttggtgacgtcgg</u> <u>ggctgacaccctgggtcatatc</u>***gca***<u>gaagcttgtgccaaaggcgaagctg</u>ataacggtcgtaaaggcccgctcaatc tgccaaatctgacccgtctggggctggcgaaagctcacgaaggttctaccggtttcattccggcgggaatggacggc aacgctgaagttatcggcgcgtacgcatgggcgcacgaaatgtcatccggtaaagatagcgtgtctggtcactggga aattgccggtgtcccggttctgtttgagtggggatatttctccgatcacgaaaacagcttcccgcaagagctgctggata aactggtcgaacgcgctaatctgccgggttacctcggtaactgccgttcttccggtacggtcattctggatcaactgggc gaagagcacatgaaaaccggcaagtggattttctatacctccgctgcatccgtgttccagattgcctgccatgaagaaa ctttcggtctggataaactctacgaactgtgcgaaatcgcccgtgaagagctgaccaacggcggctacaatatcggtc gtgttatcgctcgtccgtttatcggcgacaaagccggtaacttccagcgtaccggtaaccgtcgtgacctggctgttgag ccgccagcaccgaccgtgctgcagaaactggttgatgaaaaacacggccaggtggtttctgtcggtaaaattgcgga catctacgccaactgcggtatcaccaaaaaagtgaaagcgactggcctggacgcgctgtttgacgccaccatcaaag agatgaaagaagcgggtgataacaccatcgtcttcaccaacttcgttgacttcgactcttcctgggggccaccgtcgcga cgtcgccggttatgccgcgggtctggaactgttcgaccgccgtctgccggagctgatgtctctgctgcgcgatgacga catcctgatcctcaccgctgaccacggttgcgatccgacctggaccggtactgaccacacgcgtgaacacattccggt actggtatatggcccgaaagtaaaaccgggctcactgggtcatcgtgaaaccttcgcggatatcggccagactctggc aaaatattttggtacttctgatatggaatatggcaaagccatgttctaa (SEQ ID NO: 2)

Genscript was commissioned to use a chip to synthesize a pool of oligonucleotide sequences comprising mutant nucleotides. Each oligonucleotide was a nucleotide sequence encoding a protein comprising a mutant amino acid of one of the other 19 common amino acids at position 35. Each oligonucleotide is 80 bp in length. Particularly, the ratio of each amino acid mutation is 1/19. The sequences are as follows:

agaagatgcagaacgctttggtgacgtcggggctgacaccctgggtcatatcGGCgaagcttgtgccaaaggcg aagctg (SEQ ID NO: 3);

agaagatgcagaacgctttggtgacgtcggggctgacaccctgggtcatatcGTTgaagcttgtgccaaaggcg

aagctg (SEQ ID NO: 4);

agaagatgcagaacgctttggtgacgtcggggctgacaccctgggtcatatcCTGgaagcttgtgccaaaggcg aagctg (SEQ ID NO: 5);

agaagatgcagaacgctttggtgacgtcggggctgacaccctgggtcatatcATTgaagcttgtgccaaaggcg aagctg (SEQ ID NO: 6);

agaagatgcagaacgctttggtgacgtcggggctgacaccctgggtcatatcATGgaagcttgtgccaaaggcg aagctg (SEQ ID NO: 7);

agaagatgcagaacgctttggtgacgtcggggctgacaccctgggtcatatcTTTgaagcttgtgccaaaggcg aagctg (SEQ ID NO: 8);

agaagatgcagaacgctttggtgacgtcggggctgacaccctgggtcatatcTGGgaagcttgtgccaaaggcg aagctg (SEQ ID NO: 9);

agaagatgcagaacgctttggtgacgtcggggctgacaccctgggtcatatcCCGgaagcttgtgccaaaggcg aagctg (SEQ ID NO: 10);

agaagatgcagaacgctttggtgacgtcggggctgacaccctgggtcatatcTCAgaagcttgtgccaaaggcg aagctg (SEQ ID NO: 11);

agaagatgcagaacgctttggtgacgtcggggctgacaccctgggtcatatcACAgaagcttgtgccaaaggcg aagctg (SEQ ID NO: 12);

agaagatgcagaacgctttggtgacgtcggggctgacaccctgggtcatatcTGCgaagcttgtgccaaaggcg aagctg (SEQ ID NO: 13);

agaagatgcagaacgctttggtgacgtcggggctgacaccctgggtcatatcTATgaagcttgtgccaaaggcg aagctg (SEQ ID NO: 14);

agaagatgcagaacgctttggtgacgtcggggctgacaccctgggtcatatcAATgaagcttgtgccaaaggcg aagctg (SEQ ID NO: 15);

agaagatgcagaacgctttggtgacgtcggggctgacaccctgggtcatatcCAAgaagcttgtgccaaaggcg aagctg (SEQ ID NO: 16);

agaagatgcagaacgctttggtgacgtcggggctgacaccctgggtcatatcGATgaagcttgtgccaaaggcg aagctg (SEQ ID NO: 17);

agaagatgcagaacgctttggtgacgtcggggctgacaccctgggtcatatcGAAgaagcttgtgccaaaggcg aagctg (SEQ ID NO: 18)

agaagatgcagaacgctttggtgacgtcggggctgacaccctgggtcatatcAAAgaagcttgtgccaaaggcg aagctg (SEQ ID NO: 19);

agaagatgcagaacgctttggtgacgtcggggctgacaccctgggtcatatcAGAgaagcttgtgccaaaggcg aagctg (SEQ ID NO: 20); and

agaagatgcagaacgctttggtgacgtcggggctgacaccctgggtcatatcCATgaagcttgtgccaaaggcg aagctg (SEQ ID NO: 21).

**Example 2. Amplification of oligonucleotides synthesized by chip**

The amplification primers were designed, wherein the forward primer was named Chip-F, and the reverse primer was named Chip-R. The primer sequences are as follows: Chip-F: agaagatgcagaacgctttggtgac (SEQ ID NO: 22), and the reverse primer sequence is as follows: Chip-R: cagcttcgcctttggcacaagcttc (SEQ ID NO: 23). A PCR reaction system was prepared (see Table 1), wherein the template was the pool of the oligonucleotide sequences synthesized by the chip, the primers were the above-mentioned Chip-F/Chip-R, and the reaction system also comprised Phusion DNA polymerase, dNTPs and 5×HF buffer from kit Phusion® High-Fidelity DNA Polymerase (purchased from New England Biolabs), and water were added to a total volume of 50 μl. PCR amplification was performed on the oligonucleotides synthesized by chip according to the PCR reaction procedure shown in Table 2. The amplification results are shown in Fig. 2.

Table 1:

| HF buffer (5x) | 10 μl |
|---|---|
| 10 nM dNTP | 1 μl |
| Template (oligonucleotide synthesized by chip) | 100 ng |
| Forward primer: Chip-F | 2 μl |
| Reverse primer: Chip-R | 2 μl |
| BSA (20 mg/ml) | 10 μl |
| Phusion (2 U/μl) | 0.5 μl |
| $H_2O$ | Added to 50 μl |

The PCR reaction procedure is shown in Table 2:

Table 2:

| 1 | 98°C 30 seconds |
|---|---|
| 2 | 98°C 10 seconds |
| 3 | 64°C 10 seconds |
| 4 | 72°C 20 seconds |
| 5 | return to step 2, 16 cycles |
| 6 | 72°C 5 minutes |

**Example 3 Linear amplification using amplified oligonucleotides synthesized by the chip as primers**

PCR amplification was performed by using the plasmid PMB1 extracted from *E. coli* TOP10 comprising the full-length gene to be mutated and the methylated site as a template and using the amplified oligonucleotides synthesized by the chip in Example 2 as primers. The amplified oligonucleotides synthesized by the chip in Example 2 were double-stranded DNAs, both strands can be used as primers to amplify towards the front and rear ends respectively, and both amplifications were linear amplifications. The number of primers required for linear amplification was much smaller than that for exponential amplification. Therefore, during linear amplification, it is necessary to strictly control the ratio of primers (that is, the amplified oligonucleotides synthesized by the chip in Example 2) to templates (that is, plasmids) in the system. The molar ratio of the plasmid templates to the amplified oligonucleotides synthesized by the chip (double-stranded) in Example 2 was 1 : 20, and the cycle number of the PCR reaction was 15. The PCR reaction system is shown in Table 3:

Table 3

| HF buffer (5x) | 10 $\mu$l |
|---|---|
| 10 nM dNTP | 1 $\mu$l |
| Template (PMB1) | 1.5 ng |
| Oligonucleotide synthesized by chip (dsDNA) | 0.473 ng |
| Phusion DNA polymerase (2 U/$\mu$l) | 0.5 $\mu$l |
| H$_2$O | Added to 50 $\mu$l |

The PCR reaction procedure is shown in Table 4:

Table 4

| 1 | 98°C 30 seconds |
|---|---|
| 2 | 98°C 10 seconds |
| 3 | 60°C 30 seconds |
| 4 | 72°C 15 seconds |
| 5 | return to step 2, 15 cycles |
| 6 | 72°C 5 minutes |

**Example 4. Digestion of plasmid template in amplification system by *Dpn*I**

If the subsequent amplification system contains the PMB1 plasmid template, then the original full-length gene without any mutation would be generated in the next amplification reaction, therefore the plasmid in the reaction system needed to be removed. *E. coli* TOP10 strain contains Dam methylase, which can catalyze the adenine methylation of GATC in the plasmid to form G$^m$ATC. Therefore, the plasmid PMB1 extracted from *E. coli* DH5$\alpha$ contains methylation modification. *Dpn*I can recognize and cleave the methylated site G$^m$ATC. The plasmid templates in the amplification system were digested into small fragments by adding *Dpn*I and cannot be used as the template for the next PCR amplification. The preparation of enzyme digestion system comprised adding 10 $\mu$l of PCR reaction product, *Dpn*I (New England Biolabs, R0176L) and 10 × Cutsamrt, and adding water to a total volume of 50 $\mu$l. The reaction was performed at the optimum temperature for *Dpn*I enzyme, 37°C, for 2 hours.

The enzyme digestion system is shown in Table 5:

Table 5

| PCR product obtained in Example 3 | 10 $\mu$l |
|---|---|
| Cutsamrt | 5 $\mu$l |
| *Dpn*I | 1 $\mu$l |
| H$_2$O | Added to 50 $\mu$l |

**Example 5. PCR amplification to obtain final product**

The head and tail primers of the gene were designed. The forward primer was set at the head of the gene and named Gene-F, and the reverse primer was set at the tail of the gene and named Gene-R. The primer sequences are as follows: Gene-F: atgaaacgtgcatttattatgg (SEQ ID NO: 24), and Gene-R: ttagaacatggctttgccatat (SEQ ID NO: 25). The preparation of the PCR reaction system comprised using the product system digested with the endonuclease *Dpn*I obtained in Example 4 as a template, and adding primers Gene-F/Gene-R, Phusion DNA polymerase, dNTP, and 5xHF buffer to the reaction system, and adding water to a total volume of 50 μl to amplify the full-length sequence of the gene. The sense and antisense PCR amplification products (that were two single-stranded DNAs, which had complementary nucleosides at the mutation sites) obtained by respectively using the two strands of the oligonucleotides synthesized by chip as primers in Example 3 can be linked together by complementary regions and used as a template for the PCR amplification reaction. After PCR was completed, the product was subjected to agarose gel electrophoresis. The band corresponding to the size of the final product was subjected to gel cutting, and recovered and purified by using axygen DNA gel recovery kit (purchased from AXYGEN, article number: AP-GX-250) to obtain the final product, as shown in Fig. 3.

The reaction system of the PCR amplification is shown in Table 6:

Table 6

| | |
|---|---|
| HF buffer (5x) | 10 μl |
| 10 nM dNTP | 1 μl |
| Template | 2.5 μl |
| Forward primer Gene-F | 1 μl |
| Reverse primer Gene-R | 1 μl |
| Phusion (2 U/μl) | 0.5 μl |
| $H_2O$ | Added to 50 μl |

The reaction procedure of the PCR amplification reaction is shown in Table 7:

Table 7

| | |
|---|---|
| 1 | 98°C 30 seconds |
| 2 | 98°C 10 seconds |
| 3 | 60°C 30 seconds |
| 4 | 72°C 20 seconds |
| 5 | return to step 2, 15 cycles |
| 6 | 72°C 5 minutes |

**Example 6. Identification of final product**

The DNA recovered and purified in Example 5 was subjected to Sanger sequencing, and the sequencing results are shown in Figures 4A-4C. Fig. 4A shows that at the target mutation sites, *i.e.*, at the codon positions of amino acid at position 35 (*i.e.*, at the nucleotides at positions 103-105), a mutation occurred.

The DNA recovered and purified in Example 5 was subjected to high-throughput sequencing, and the mutant nucleotide sequences at the mutation sites and the frequency of each mutant nucleotide sequence in the obtained DNA sequence were analyzed. The high-throughput sequencing results are shown in Table 8.

Table 8

| | |
|---|---|
| Expected oligonucleotide species | 19 |
| Detected oligonucleotide species | 19 |
| Max sequencing depth | 284 |
| Average sequencing depth | 230.85 |

(continued)

| | |
|---|---|
| Sequencing depth ranked 10% | 211 |
| Sequencing depth ranked 90% | 258 |
| Deviation degree (depth ranked 90%/10% ) | 1.22 |

**Example 7. Comparison of different ratios of templates to primers in the linear amplification step using the oligonucleotides synthesized by chip as primers**

Different ratios of templates to primers in the linear amplification step using the oligonucleotides synthesized by chip as primers were tested. PCR amplification was performed by using the plasmid PMB1 extracted from *E. coli* TOP10 comprising the full-length gene to be mutated and the methylated site as a template and using the amplified oligonucleotides synthesized by the chip in Example 2 as primers. The molar ratio of the template plasmids to the amplified oligonucleotides synthesized by the chip (double-stranded) in Example 2 was 1 : 254, and the cycle number of the PCR reaction was 25. The PCR reaction system is shown in Table 8, and the reaction procedure is shown in Table 9. Then the same steps as Examples 4-6 were repeated, and the electrophoresis result of the finally obtained full-length gene is shown in Fig. 5, wherein the band is shallow and strongly smeared.

Table 8

| | |
|---|---|
| HF buffer (5x) | 10 $\mu$l |
| 10 nM dNTP | 1 $\mu$l |
| Template (PMB1) | 20 ng |
| Oligonucleotide synthesized by chip (dsDNA) | 80 ng |
| Phusion DNA polymerase (2 U/$\mu$l) | 0.5 $\mu$l |
| H$_2$O | Added to 50 $\mu$l |

The PCR reaction procedure is shown in Table 9:

Table 9

| | |
|---|---|
| 1 | 98°C 30 seconds |
| 2 | 98°C 10 seconds |
| 3 | 60°C 30 seconds |
| 4 | 72°C 15 seconds |
| 5 | return to step 2, 25 cycles |
| 6 | 72°C 5 minutes |

The cycle number of the reaction was optimized. PCR amplification was performed by using the plasmid PMB1 extracted from *E. coli* TOP10 comprising the full-length gene to be mutated and the methylated site as a template and using the amplified oligonucleotides synthesized by the chip in Example 2 as primers. The molar ratio of the template plasmids to the amplified oligonucleotides synthesized by the chip (double-stranded) in Example 2 was 1 : 254, and the cycle number of the PCR reaction was reduced to 15. The PCR reaction system is shown in Table 8, and the reaction procedure is shown in Table 10. The electrophoresis result of the finally obtained full-length gene is shown in Fig. 6, wherein the band is shallow and slightly smeared.

Table 10

| | |
|---|---|
| 1 | 98°C 30 seconds |
| 2 | 98°C 10 seconds |
| 3 | 60°C 30 seconds |

(continued)

| 4 | 72°C 15 seconds |
|---|---|
| 5 | return to step 2, 15 cycles |
| 6 | 72°C 5 minutes |

**Example 8: Comparison between the method for constructing a gene mutation library of the present invention and the method for constructing a gene mutation library in the prior art**

Conventional method for constructing a gene mutation library in the prior art are: 1. amplifying a mutant region synthesized by a chip by PCR, and then performing column purification, 2. amplifying the non-mutant regions before and after the mutant region using a gene to be mutated as a template, wherein the region in front of the mutant region and the region behind the mutant region need to be amplified separately and are respectively subjected to a PCR reaction, then performing agarose gel electrophoresis, and recovering and purifying DNA fragments by cutting the gel; 3. assembling the 3 fragments into one fragment by assembly PCR, and 4. amplifying a full-length fragment by using the assembled product as a template (without purification) and adding head and tail primers for gene, then performing agarose gel electrophoresis, and recovering and purifying DNA fragments by cutting the gel.

The above conventional method for constructing a gene mutation library in the prior art takes about 6.5 hours in total, while the method for constructing a gene mutation library of the present invention takes about 5.5 hours. It can be seen that the method for constructing a gene mutation library of the present invention has fewer steps and saves time.

It should also be noted that, on the premise that it can be implemented and does not obviously violate the gist of the present invention, any technical feature or combination of technical features described as a constituent part of a technical solution in the present description can also be applied to other technical solutions; in addition, on the premise that it can be implemented and does not obviously violate the gist of the present invention, the technical features described as constituent parts of different technical solutions can also be combined in any way to form other technical solutions. The present invention also comprises technical solutions obtained by combining in the above-mentioned cases, and these technical solutions are equivalent to being described in the present invention.

The present invention was described above by specific embodiments and examples, but those skilled in the art should understand that these are not intended to limit the scope of the present invention. The scope of the present invention should be determined by the claims.

**Claims**

1. A method for constructing a gene mutation library, **characterized in that** the method comprises

(1) synthesizing a pool of oligonucleotide sequences comprising mutant nucleotide(s) at one or more mutation sites;
(2) performing PCR amplification on the oligonucleotides in the synthesized pool of the oligonucleotide sequences comprising the mutant nucleotide(s);
(3) performing PCR amplification by using the plasmid comprising a methylated site and a gene to be mutated or a part thereof as a template and using the oligonucleotides in the pool of the amplified oligonucleotide sequences obtained in step (2) as primers;
(4) using an endonuclease that recognizes and cleaves the methylated site to cleave the template plasmid present in the amplified system of step (3); and
(5) adding a forward primer and a reverse primer respectively corresponding to both ends of the gene to be mutated or the part thereof to the system obtained in step (4) and performing PCR amplification to obtain the gene mutation library comprising a plurality of genes containing the mutant nucleotide(s) or a part thereof.

2. The method according to claim 1, **characterized in that** purification is not performed during steps (1)-(5).

3. The method according to claim 1, **characterized in that** the method further comprises
(6) recovering and purifying the amplified product of step (5) to obtain a final product.

4. The method according to claim 3, **characterized in that** the method further comprises
(7) sequencing the final product obtained in step (6) to verify sequence and/or detect gene mutation distribution.

5. The method according to claim 4, **characterized in that** the sequencing is Sanger sequencing and/or high-throughput sequencing.

6. The method according to any one of claims 1-5, **characterized in that** the length of the oligonucleotide sequences in the pool of the oligonucleotide sequences synthesized in step (1) is 60-170 bp, preferably 80-90 bp.

7. The method according to any one of claims 1-6, **characterized in that** the pool of the oligonucleotide sequences synthesized in step (1) comprises mutant nucleotide(s) at 1 to 10 mutation sites.

8. The method according to any one of claims 1-7, **characterized in that** the mutation sites are either adjacent or non-adjacent.

9. The method according to any one of claims 1-8, **characterized in that** the amino acid sequence encoded by a gene comprising the mutant nucleotide(s) has at least one amino acid difference compared to the amino acid sequence encoded by the gene to be mutated.

10. The method according to claim 9, **characterized in that** the at least one amino acid difference is selected from: substitution, addition or deletion.

11. The method according to claim 10, **characterized in that** the at least one amino acid difference is substitution.

12. The method according to claim 11, **characterized in that** the substitution is to substitute the original amino acid with an amino acid having different properties from the original amino acid.

13. The method according to claim 12, **characterized in that** the properties are selected from: acidity and alkalinity, polarity, charge characteristic and side chain group.

14. The method according to any one of claims 1-13, **characterized in that** two primers respectively corresponding to both ends of the oligonucleotide sequences comprising the mutant nucleotide(s) at one or more mutation sites are used in the PCR amplification in step (2).

15. The method according to any one of claims 1-14, **characterized in that** the number of cycles of the PCR amplification in step (2) is 10-25 cycles, preferably 12-22 cycles, 14-20 cycles, 15-18 cycles or 15-16 cycles, and most preferably 16 cycles.

16. The method according to any one of claims 1-15, **characterized in that** the DNA polymerase used in the PCR amplification in step (2) is a high-fidelity DNA polymerase.

17. The method according to any one of claims 1-16, **characterized in that** the molar ratio of the primers to the template in step (3) is 15 : 1 to 50 : 1, preferably 16 : 1 to 40 : 1, 17 : 1 to 30 : 1, 18 : 1 to 25 : 1, or 19 : 1 to 22 : 1, more preferably 19 : 1 to 21 : 1, and most preferably 20 : 1.

18. The method according to any one of claims 1-17, **characterized in that** the number of cycles of the PCR amplification in step (3) is 10-20 cycles, preferably 12-18 cycles, 14-16 cycles, and most preferably 15 cycles.

19. The method according to any one of claims 1-18, **characterized in that** the DNA polymerase used in the PCR amplification in step (3) is a high-fidelity DNA polymerase, preferably a high-fidelity Phusion DNA polymerase.

20. The method according to any one of claims 1-19, **characterized in that** the plasmid is extracted from bacteria that methylate the plasmid, preferably from *E. coli,* more preferably from *E. coli* TOP10 strain.

21. The method according to any one of claims 1-20, **characterized in that** the endonuclease in step (4) is *Dpn*I, *Msp*JI or *Fsp*EI.

22. A gene mutation library constructed using the method according to any one of claims 1-21.

23. A kit for the method for constructing the gene mutation library according to any one of claims 1-21, **characterized in that** the kit comprises a pool of oligonucleotide sequences comprising mutant nucleotide(s) at one or more mutation sites, a plasmid comprising a methylated site and a gene to be mutated or a part thereof, and an endonuclease that recognizes and cleaves the methylated site.

**24.** The kit according to claim 23, **characterized in that** the kit further comprises a DNA polymerase.

**25.** A method for analyzing the relationship between an amino acid mutation in a protein and the properties, regulation and/or function of the protein, **characterized in that** the method comprises the following steps:

(1) constructing a gene mutation library of a gene encoding the protein using the method according to any one of claims 1-21;
(2) comparing the properties, regulation and/or function of the protein encoded by a mutant gene in the gene mutation library with the properties, regulation and/or function of the unmutated protein; and
(3) analyzing the relationship between the amino acid at the mutation position and the properties, regulation and/or function of the protein.

Chip oligonucleotide
fragment

Amino acid
of interest

Amplification using chip
oligonucleotide

Digestion by
Dpnl enzyme

Amplification for
final product

*Fig. 1*

250bp
100bp

*Fig. 2*

3000bp
2000bp
1500bp
1000bp

*Fig. 3*

Fig. 4A

Project: Untitled Contig 1

```
                    10        20        30        40        50        60        70        80
          ....|....|....|....|....|....|....|....|....|....|....|....|....|....|....|....|....|....
          ATGAAACGTGCATTTATTATGGTGCTGGACTCATTCGGCATCGGCGCTACAGAAGATGCAGAACGCTTTGGTGACGTCGGGGCTGA

TEMPLATE.SEQ(1>1224)    →   atgaaacgtgcatttattatggtgctggactcattcggcatcggcgctacagaagatgcagaacgctttggtgacgtcggggctga
L35-1_48.ab1(13>899)   →   ATGAAACGTGCATTTATTATGGTGCTGGACTCATTCGGCATCGGCGCTACAGAAGATGCAGAACGCTTTGGTGACGTCGGGGCTGA


               90       100       110       120       130       140       150       160       170
          ....|....|....|....|....|....|....|....|....|....|....|....|....|....|....|....|....|....
          CACCCTGGGTCATATCGCAGAAGCTTGTGCCAAAGGCGAAGCTGATAACGGTCGTAAAGGCCCGCTCAATCTGCCAAATCTGACCC

TEMPLATE.SEQ(1>1224)    →   caccctgggtcatatcgcagaagcttgtgccaaaggcgaagctgataacggtcgtaaaggcccgctcaatctgccaaatctgaccc
L35-1_48.ab1(13>899)   →   CACCCTGGGTCATATCAGAGAAGCTTGTGCCAAAGGCGAAGCTGATAACGGTCGTAAAGGCCCGCTCAATCTGCCAAATCTGACCC


              180       190       200       210       220       230       240       250
          ....|....|....|....|....|....|....|....|....|....|....|....|....|....|....|....|....|....
          GTCTGGGGCTGGCGAAAGCTCACGAAGGTTCTACCGGTTTCATTCCGGCGGGAATGGACGGCAACGCTGAAGTTATCGGCGCGTAC

TEMPLATE.SEQ(1>1224)    →   gtctggggctggcgaaagctcacgaaggttctaccggtttcattccggcgggaatggacggcaacgctgaagttatcggcgcgtac
L35-1_48.ab1(13>899)   →   GTCTGGGGCTGGCGAAAGCTCACGAAGGTTCTACCGGTTTCATTCCGGCGGGAATGGACGGCAACGCTGAAGTTATCGGCGCGTAC


          260       270       280       290       300       310       320       330       340
          ....|....|....|....|....|....|....|....|....|....|....|....|....|....|....|....|....|....
          GCATGGGCGCACGAAATGTCATCCGGTAAAGATAGCGTGTCTGGTCACTGGGAAATTGCCGGTGTCCCGGTTCTGTTTGAGTGGGG

TEMPLATE.SEQ(1>1224)    →   gcatgggcgcacgaaatgtcatccggtaaagatagcgtgtctggtcactgggaaattgccggtgtcccggttctgtttgagtgggg
L35-1_48.ab1(13>899)   →   GCATGGGCGCACGAAATGTCATCCGGTAAAGATAGCGTGTCTGGTCACTGGGAAATTGCCGGTGTCCCGGTTCTGTTTGAGTGGGG


             350       360       370       380       390       400       410       420       430
          ....|....|....|....|....|....|....|....|....|....|....|....|....|....|....|....|....|....
          ATATTTCTCCGATCACGAAAACAGCTTCCCGCAAGAGCTGCTGGATAAACTGGTCGAACGCGCTAATCTGCCGGGTTACCTCGGTA

TEMPLATE.SEQ(1>1224)    →   atatttctccgatcacgaaaacagcttcccgcaagagctgctggataaactggtcgaacgcgctaatctgccgggttacctcggta
L35-1_48.ab1(13>899)   →   ATATTTCTCCGATCACGAAAACAGCTTCCCGCAAGAGCTGCTGGATAAACTGGTCGAACGCGCTAATCTGCCGGGTTACCTCGGTA
```

EP 4 086 344 A1

EP 4 086 344 A1

```
                   440        450        460        470        480        490        500        510
        ..|....|....|....|....|....|....|....|....|....|....|....|....|....|....|....|....|....|....|....|
        ACTGCCGTTCTTCCGGTACGGTCATTCTGGATCAACTGGGCGAAGAGCACATGAAAACCGGCAAGTGGATTTTCTATACCTCCGCT

TEMPLATE.SEQ(1>1224)    →    actgccgttcttccggtacggtcattctggatcaactgggcgaagagcacatgaaaaccggcaagtggattttctatacctccgct
L35-1_48.ab1(13>899)   →    ACTGCCGTTCTTCCGGTACGGTCATTCTGGATCAACTGGGCGAAGAGCACATGAAAACCGGCAAGTGGATTTTCTATACCTCCGCT
```

```
                   520        530        540        550        560        570        580        590        600
        ..|....|....|....|....|....|....|....|....|....|....|....|....|....|....|....|....|....|....|....|
        GCATCCGTGTTCCAGATTGCCTGCCATGAAGAAACTTTCGGTCTGGATAAACTCTACGAACTGTGCGAAATCGCCCGTGAAGAGCT

TEMPLATE.SEQ(1>1224)    →    gcatccgtgttccagattgcctgccatgaagaaactttcggtctggataaactctacgaactgtgcgaaatcgcccgtgaagagct
L35-1_48.ab1(13>899)   →    GCATCCGTGTTCCAGATTGCCTGCCATGAAGAAACTTTCGGTCTGGATAAACTCT
L35-1-47.ab1(23>847)   ←                                                            ACGAACTGTGCGAAATCGCCCGTGAAGAGCT
```

```
                   610        620        630        640        650        660        670        680
        ..|....|....|....|....|....|....|....|....|....|....|....|....|....|....|....|....|....|....|....|
        GACCAACGGCGGCTACAATATCGGTCGTGTTATCGCTCGTCCGTTTATCGGCGACAAAGCCGGTAACTTCCAGCGTACCGGTAACC

TEMPLATE.SEQ(1>1224)    →    gaccaacggcggctacaatatcggtcgtgttatcgctcgtccgtttatcggcgacaaagccggtaacttccagcgtaccggtaacc
L35-1-47.ab1(23>847)   ←    GACCAACGGCGGCTACAATATCGGTCGTGTTATCGCTCGTCCGTTTATCGGCGACAAAGCCGGTAACTTCCAGCGTACCGGTAACC
```

```
                   690        700        710        720        730        740        750        760        770
        ..|....|....|....|....|....|....|....|....|....|....|....|....|....|....|....|....|....|....|....|
        GTCGTGACCTGGCTGTTGAGCCGCCAGCACCGACCGTGCTGCAGAAACTGGTTGATGAAAAACACGGCCAGGTGGTTTCTGTCGGT

TEMPLATE.SEQ(1>1224)    →    gtcgtgacctggctgttgagccgccagcaccgaccgtgctgcagaaactggttgatgaaaaacacggccaggtggtttctgtcggt
L35-1-47.ab1(23>847)   ←    GTCGTGACCTGGCTGTTGAGCCGCCAGCACCGACCGTGCTGCAGAAACTGGTTGATGAAAAACACGGCCAGGTGGTTTCTGTCGGT
```

```
                   780        790        800        810        820        830        840        850        860
        ..|....|....|....|....|....|....|....|....|....|....|....|....|....|....|....|....|....|....|....|
        AAAATTGCGGACATCTACGCCAACTGCGGTATCACCAAAAAAGTGAAAGCGACTGGCCTGGACGCGCTGTTTGACGCCACCATCAA

TEMPLATE.SEQ(1>1224)    →    aaaattgcggacatctacgccaactgcggtatcaccaaaaaagtgaaagcgactggcctggacgcgctgtttgacgccaccatcaa
L35-1-47.ab1(23>847)   ←    AAAATTGCGGACATCTACGCCAACTGCGGTATCACCAAAAAAGTGAAAGCGACTGGCCTGGACGCGCTGTTTGACGCCACCATCAA
```

```
                   870        880        890        900        910        920        930        940
        ..|....|....|....|....|....|....|....|....|....|....|....|....|....|....|....|....|....|....|....|
        AGAGATGAAAGAAGCGGGTGATAACACCATCGTCTTCACCAACTTCGTTGACTTCGACTCTTCCTGGGGCCACCGTCGCGACGTCG

TEMPLATE.SEQ(1>1224)    →    agagatgaaagaagcgggtgataacaccatcgtcttcaccaacttcgttgacttcgactcttcctggggccaccgtcgcgacgtcg
L35-1-47.ab1(23>847)   ←    AGAGATGAAAGAAGCGGGTGATAACACCATCGTCTTCACCAACTTCGTTGACTTCGACTCTTCCTGGGGCCACCGTCGCGACGTCG
```

*Fig. 4B*

```
              950       960       970       980       990       1000      1010      1020      1030
              ..|....|....|....|....|....|....|....|....|....|....|....|....|....|....|....|....|....|
              CCGGTTATGCCGCGGGTCTGGAACTGTTCGACCGCCGTCTGCCGGAGCTGATGTCTCTGCTGCGCGATGACGACATCCTGATCCTC

TEMPLATE.SEQ(1>1224)   ─────▶  ccggttatgccgcgggtctggaactgttcgaccgccgtctgccggagctgatgtctctgctgcgcgatgacgacatcctgatcctc
L35-1-47.ab1(23>847)   ●───    CCGGTTATGCCGCGGGTCTGGAACTGTTCGACCGCCGTCTGCCGGAGCTGATGTCTCTGCTGCGCGATGACGACATCCTGATCCTC


              1040      1050      1060      1070      1080      1090      1100      1110
              ..|....|....|....|....|....|....|....|....|....|....|....|....|....|....|....|....|....|...
              ACCGCTGACCACGGTTGCGATCCGACCTGGACCGGTACTGACCACACGCGTGAACACATTCCGGTACTGGTATATGGCCCGAAAGT

TEMPLATE.SEQ(1>1224)   ─────▶  accgctgaccacggttgcgatccgacctggaccggtactgaccacacgcgtgaacacattccggtactggtatatggcccgaaagt
L35-1-47.ab1(23>847)   ●───    ACCGCTGACCACGGTTGCGATCCGACCTGGACCGGTACTGACCACACGCGTGAACACATTCCGGTACTGGTATATGGCCCGAAAGT


              1120      1130      1140      1150      1160      1170      1180      1190      1200
              .|....|....|....|....|....|....|....|....|....|....|....|....|....|....|....|....|....|....
              AAAACCGGGCTCACTGGGTCATCGTGAAACCTTCGCGGATATCGGCCAGACTCTGGCAAAATATTTTGGTACTTCTGATATGGAAT

TEMPLATE.SEQ(1>1224)   ─────▶  aaaaccgggctcactgggtcatcgtgaaaccttcgcggatatcggccagactctggcaaaatatttttggtacttctgatatggaat
L35-1-47.ab1(23>847)   ●───    AAAACCGGGCTCACTGGGTCATCGTGAAACCTTCGCGGATATCGGCCAGACTCTGGCAAAATATTTTGGTACTTCTGATATGGAAT


              1210      1220
              |....|....|....|....
              ATGGCAAAGCCATGTTCTAA

TEMPLATE.SEQ(1>1224)   ─────▶  atggcaaagccatgttctaa
L35-1-47.ab1(23>847)   ●───    ATGGCAAAGCCATGTTCTAA
```

*Fig. 4C*

1500bp
1000bp

*Fig. 5*

1500bp
1000bp

*Fig. 6*

## INTERNATIONAL SEARCH REPORT

| | International application No. |
|---|---|
| | **PCT/CN2020/140247** |

**A. CLASSIFICATION OF SUBJECT MATTER**

C12N 15/10(2006.01)i; C40B 50/06(2006.01)i

According to International Patent Classification (IPC) or to both national classification and IPC

**B. FIELDS SEARCHED**

Minimum documentation searched (classification system followed by classification symbols)

C12N; C40B

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, USTXT, EPTXT, CNKI, 万方数据资源系统, PubMed, ISI web of knowledge: 南京金斯瑞生物科技有限公司, 构建, 突变, 文库, 甲基化, 寡核苷酸, 内切酶, Phusion DNA聚合酶, DpnI, MspJI, FspEI, mutant, mutation, library, pool, methylation

**C. DOCUMENTS CONSIDERED TO BE RELEVANT**

| Category* | Citation of document, with indication, where appropriate, of the relevant passages | Relevant to claim No. |
|---|---|---|
| X | CN 108424907 A (PEKING UNIVERSITY) 21 August 2018 (2018-08-21)<br>claim 1, and description, paragraph [0008] | 1-25 |
| A | CN 110305861 A (SHENZHEN FEIPENG BIOLOGICAL TREATMENT CO., LTD.) 08 October 2019 (2019-10-08)<br>entire document | 1-25 |
| A | CN 109763172 A (BGI RESEARCH) 17 May 2019 (2019-05-17)<br>entire document | 1-25 |
| A | CN 101580829 A (SHENZHEN UNIVERSITY) 18 November 2009 (2009-11-18)<br>entire document | 1-25 |
| A | CN 108796619 A (CATHAY BIOTECH INC. et al.) 13 November 2018 (2018-11-13)<br>entire document | 1-25 |
| A | CN 109750032 A (SYNCOZYMES (SHANGHAI) CO., LTD.) 14 May 2019 (2019-05-14)<br>entire document | 1-25 |
| A | CN 104877977 A (JIANGNAN UNIVERSITY) 02 September 2015 (2015-09-02)<br>entire document | 1-25 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **05 March 2021** | **25 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2020/140247** |

**C.      DOCUMENTS CONSIDERED TO BE RELEVANT**

| Category* | Citation of document, with indication, where appropriate, of the relevant passages | Relevant to claim No. |
| --- | --- | --- |
| A | WO 2018183612 A1 (EDITAS MEDICINE, INC.) 04 October 2018 (2018-10-04)<br>    entire document | 1-25 |
| A | EP 1544296 A1 (BIOMETHODES) 22 June 2005 (2005-06-22)<br>    entire document | 1-25 |

Form PCT/ISA/210 (second sheet) (January 2015)

**INTERNATIONAL SEARCH REPORT**

| International application No. |
| --- |
| **PCT/CN2020/140247** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.   ☑  forming part of the international application as filed:

           ☑   in the form of an Annex C/ST.25 text file.

           ☐   on paper or in the form of an image file.

    b.   ☐  furnished together with the international application under PCT Rule 13*ter*.1(a) for the purposes of international search only in the form of an Annex C/ST.25 text file.

    c.   ☐  furnished subsequent to the international filing date for the purposes of international search only:

           ☐   in the form of an Annex C/ST.25 text file (Rule 13*ter*.1(a)).

           ☐   on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/CN2020/140247**

| Patent document cited in search report | | | Publication date (day/month/year) | Patent family member(s) | | | Publication date (day/month/year) |
|---|---|---|---|---|---|---|---|
| CN | 108424907 | A | 21 August 2018 | None | | | |
| CN | 110305861 | A | 08 October 2019 | None | | | |
| CN | 109763172 | A | 17 May 2019 | None | | | |
| CN | 101580829 | A | 18 November 2009 | CN | 101580829 | B | 18 May 2011 |
| CN | 108796619 | A | 13 November 2018 | None | | | |
| CN | 109750032 | A | 14 May 2019 | None | | | |
| CN | 104877977 | A | 02 September 2015 | None | | | |
| WO | 2018183612 | A1 | 04 October 2018 | US | 2020181666 | A1 | 11 June 2020 |
| EP | 1544296 | A1 | 22 June 2005 | AT | 428779 | T | 15 May 2009 |
| | | | | EP | 1544296 | B1 | 15 April 2009 |
| | | | | US | 2005153343 | A1 | 14 July 2005 |
| | | | | EP | 1544296 | B8 | 05 August 2009 |
| | | | | DE | 602004020570 | D1 | 28 May 2009 |

Form PCT/ISA/210 (patent family annex) (January 2015)